# FASCICULE DE BREVET EUROPEEN

(11) **EP 3 187 864 B1**
(45) Date de publication et mention de la délivrance du brevet: **26.02.2020**
(21) Numéro de dépôt: 16207409.0
(22) Date de dépôt: 29.12.2016
(51) Int. Cl.: G01N 27/04, A01G 9/02, A01G 27/00, A01G 27/06, G01N 33/24

(54) **DISPOSITIF DE MESURE RÉSISTIVE DE L'HUMIDITÉ D'UN MATÉRIAU MINÉRAL POREUX, ET APPLICATION AU CONTRÔLE HYDRIQUE DES PLANTES**
VORRICHTUNG ZUR RESISTIVEN MESSUNG DER FEUCHTIGKEIT EINES PORÖSEN MINERALMATERIALS, UND ANWENDUNG BEIM MONITORING DER WASSERVERSORGUNG VON PFLANZEN
DEVICE FOR RESISTIVE MEASUREMENT OF THE MOISTURE OF A POROUS MINERAL MATERIAL, AND USE FOR MONITORING THE WATERING OF PLANTS

(30) Priorité: 30.12.2015 FR 1563476
(43) Date de publication de la demande: 05.07.2017
(73) Titulaire: Lestelle, Dominique, 75005 Paris (FR)
(72) Inventeur: LESTELLE, Dominique, 75005 Paris (FR); BODEN, Robert, 91410 Dourdan (FR)
(74) Mandataire: Cabinet Camus Lebkiri

(56) Documents cités:
- DE-A1-102013 018 917
- FR-A1- 2 986 328
- FR-A1- 2 988 262
- FR-A1- 2 996 101
- US-A- 2 740 032

## Description

L'invention concerne un dispositif de mesure résistive de l'humidité d'un matériau minéral poreux M à porosité ouverte. Elle concerne aussi son application à un dispositif de mesure de l'humidité d'un matériau granulaire ou pulvérulent, ou encore à un substrat de culture de plantes dont on veut mesurer l'humidité, lorsqu'il est mis en contact et en équilibre hydrique avec ce matériau M instrumenté selon l'invention. Enfin, en plaçant ledit dispositif de mesure de l'humidité au voisinage des racines d'une plante et sur le chemin d'arrivée de l'eau, on obtient un dispositif de monitoring de la consommation hydrique d'une plante.

Les dispositifs de mesure résistive de l'humidité d'un matériau M poreux sont connus depuis longtemps pour leur simplicité et leur faible coût. On rappelle que dans le cas de la mesure de l'humidité des matériaux granulaires ou pulvérulents, ils fournissent une alternative peu coûteuse aux mesures capacitives. Ces dernières placent deux électrodes parallèles dans le dit matériau pour mesurer la constante diélectrique du matériau qui les sépare. Ces électrodes sont excitées par un courant alternatif de l'ordre de quelques dizaines kHz à quelques centaines de kHz. Mais un problème vient de ce que les grains de ce matériau ont aussi une certaine résistance entre lesquels existent de nombreuses résistances de contact. Le courant alternatif injecté se répartit entre le condensateur et nombre de résistances ohmiques en parallèle, sujettes à des variations brusques ou même alternatives (si vibrations).

Ainsi la technique plus simple d'une mesure résistive pourrait redevenir avantageuse à condition de résoudre le problème posé par l'existence des résistances de contacts de valeur difficilement maîtrisable. En effet, entre les deux électrodes de mesure, il n'y a en réalité pas une seule résistance (celle du matériau M) mais trois. La première est la résistance de contact entre la première électrode et ce matériau M, la seconde la résistance du matériau M et la troisième la résistance de contact entre ce matériau M et la seconde électrode. Or une résistance de contact varie considérablement selon de multiples facteurs, comme la micro rugosité locale des matériaux en jeu, l'élasticité des parties en contact (les surfaces des électrodes en contact avec le matériau M) et la force de pression entre les éléments conducteurs en contact (ici les électrodes et le matériau M).

Selon l'art antérieur FR2 986 328 du 31/01/2012 (CEMAGREF), le matériau M est une matrice de synthèse poreuse, placée en équilibre hydrique au milieu d'un substrat de culture, et dans laquelle deux électrodes sont plantées. Toutefois le dispositif décrit comporte deux inconvénients majeurs.

Le **premier inconvénient** vient de ce que le matériau de synthèse utilisé pour se mettre en équilibre hydrique comporte une distribution des diamètres de pores insuffisamment homogène. Or cette homogénéité conditionne l'aptitude à mesurer de manière homogène toutes les tensions de succion, ce qui revient à dire toutes les valeurs d'humidité. Il est en effet connu qu'à tout diamètre d'un interstice poreux (et à fortiori capillaire) correspond une pression négative de succion nécessaire pour extraire l'eau dudit interstice ou pore, comme mentionné dans le Tableau 1 ci-dessous, qui montre la relation entre la taille des pores et la tension de succion nécessaire pour les vider de leur eau. A ce tableau établi par ROWELL en 1994, ont été ajoutées par interpolation les trois valeurs (en italique et avec astérisque) mentionnées dans FR2 986 328.

**Tableau 1**

| Taille des pores (µm) | Tension de succion (hPa = mbar) | Commentaire |
|---|---|---|
| 20 000 | 0,15 | grosse crevasse |
| 4 000 | 0,75 | galerie de vers |
| 300 | 10 | diamètre d'une racine de blé |
| 60-30 | 50-100 | Tension de succion à la capacité au champ |
| *3,2* | *850** | *Céramique* |
| 2 | 1 500 | limite de pore contenant de l'eau facilement utilisable |
| *1,6* | *2 000** | *Plâtre* |
| *0,57* | *5 000** | *valeur recherchée par* FR2 986 328 |
| 0,2 | 15 000 | Point de flétrissement |
| 0,003 | 1 000 000 | Tension de succion d'un sol sec à l'air |

Cela signifie que la courbe de porosimétrie au mercure, dans la gamme de 3,2 µm à 0,57µm et si possible au-delà, doit être le plus constant possible pour que le dispositif de mesure réagisse pareillement à toutes les tensions de succion ; à défaut, il doit être monotone et avoir la pente la plus régulière possible pour permettre des corrections.

La Fig. 1 reproduit la Fig. 2 de FR2 986 328, qui « est une représentation graphique de l'intrusion incrémentale du mercure en fonction du diamètre des pores (porosimétrie au mercure) ». Le Tableau 2 récapitule pour chacun des diamètres de référence les valeurs en mL/g correspondant à la quantité de pores disponibles au diamètre considéré.

**Tableau 2**

| | diamètre des pores (µ) | tension de succion (hPa) | porosité pour matériau T1 (mL/g) | porosité pour matériau T2 (mL/g) |
|---|---|---|---|---|
| céramique | 3,2 | 850 | 0,145 (Max) | 0,008 (min) |
| plâtre+argile | 1,6 | 2 000 | 0,036 | 0,0079 (min) |
| Ø minimum | 0,57 | 5 000 | 0,07 | 0,015 |

On constate un fort pic de sensibilité pour certains diamètres de pores. Pour T1 à 4µm, 0,1 µm et 0,013µm, et pour T2 à 20µm, 0,25%m (moins marqué) et 0,013µm. Les rapports d'amplitudes peuvent être considérables, allant pour T1 d'environ 6.10⁻⁴ à 145 (soit une forte différence d'aptitude à absorber l'humidité environnante, de l'ordre de 217 000) et pour T2 de l'ordre de 0,01 à 178, soit une différence de l'ordre de 178. En outre ces courbes sont fortement non monotones, ce qui pénalise fortement toute entreprise de linéarisation.

Plus généralement, ce qui est présenté comme la matrice apte à se mettre en équilibre hydrique est réalisée synthétiquement à partir de ciment, plâtre, sable, chaux et Al. Or ces matériaux sont constitués de poudres ayant une granulométrie précise. En conséquence, les pores du matériau résultant auront eux aussi des dimensions relativement répétitives, créant un pic de sensibilité pour le diamètre de pore (et donc la tension de succion) associé à chacun des constituants. Lorsque par exemple les auteurs de FR2 986 328 augmentent la proportion d'aluminium pour étendre la plage de mesure, ils augment l'amplitude du pic correspondant au diamètre des interstices entre les grains d'aluminium. Ils obtiennent un certain prolongement de l'étendue de mesure, mais au prix d'une non linéarité croissante.

Le **second inconvénient** de FR2 986 328 vient de la manière de « disposer » les électrodes dans la matrice poreuse. Tout indique qu'elles sont insérées à force après fabrication de la matrice, préférentiellement réalisée à l'autoclave (p.3 ligne3). D'une part, la différence de conductibilité thermique entre des électrodes fortement conductrices de la chaleur et un ciment poreux assez bon isolant créerait des non homogénéités de température conduisant à la fissuration ou l'éclatement de la matrice. Et d'autre part aucun liant susceptible de réaliser un scellement n'est mentionné. Les électrodes sont donc en contact électrique avec la matrice via des résistances de contact proportionnelles aux forces de pression avec cette matrice.

Pour répondre à ces problèmes, le dispositif de mesures résistive de l'humidité selon l'invention comporte un matériau ayant une porosité constante dans une grande plage de diamètres de pores, et des électrodes qui lui sont connectées en s'affranchissant des fluctuations des résistances de contact.

### Exposé de l'invention

L'invention est définie par les revendications annexées.

Pour répondre à cela, le dispositif selon l'invention comporte un bloc d'un matériau M dans sa version de base est un dispositif comportant :
- un bloc (1) d'un matériau M, minéral, rigide et doté d'une porosité ouverte, dont on veut mesurer les variations de résistance en fonction de la teneur en eau
- au moins deux électrodes (2) et (2') de détection, rigides, inoxydables et nues, appliquées parallèlement contre ledit matériau M, et scellées sur ce dernier par une couche de liant (8) constitué d'un mélange de poudre du matériau M et de chaux chacune des électrodes (2) et (2') étant connectée à une électronique (3) apte à générer un courant i de mesure et à une électronique (4) apte à mesurer la résistance entre lesdites électrodes.

De manière préférentielle, le matériau M est un matériau naturel ayant une porosité ouverte dont la distribution des diamètres est continue entre 10 et 10 000 nm (0,01 à 10 µm). Sa courbe de porosité au mercure montre un seul pic dans cet intervalle, et de part et d'autre de ce pic elle est monotone et exempte d'extrema.

De manière encore plus préférentielle, afin d'éviter pour le matériau M les problèmes des matériau réalisés à partir de poudres, ledit matériau M est une roche tendre crayeuse ayant porosité ouverte dont la distribution des diamètres est continue entre 10 et 10000 nm (0,01 à 10 µm), dont la courbe de porosité au mercure montre un seul pic dans cet intervalle, et le liant qui scelle les électrodes au matériau M est un mélange poudre de M et de chaux, contenant entre 5 et 50% de chaux, qui assure une bonne conductivité électrique et une libre circulation de l'humidité. Il importe de mentionner que la notion de « roche tendre crayeuse » est très peu discriminante et recouvre un grand nombre de produits qui, très majoritairement n'ont pas les caractéristiques requises pour l'invention. Pour réaliser l'invention, avec un tel composant, il faut rechercher une carrière ayant les caractéristiques rares parmi les roches crayeuse et mentionnée dans la fin de la phrase, à savoir « à porosité ouverte » mais surtout « dont la distribution des diamètres est continue entre 10 et 10 000 nm (0,01 à 10 µm) », et « dont la courbe de porosité au mercure montre un seul pic dans cet intervalle, et de part et d'autre de ce pic elle est monotone et exempte d'extrema ». Ces caractéristiques sont très éloignées de la structure générale d'une roche crayeuse, mais le très grand nombre de roches crayeuses disponibles rend cette recherche relativement aisée, et les sources d'approvisionnement nombreuses. Le présent document n'a pas vocation à lister toutes les carrières de la planète produisant une roche conforme aux critères de l'invention, et chaque pays trouvera sans difficulté les siennes. Un exemple très avantageux est donné au § ci-après, qui n'est en rien limitatif.

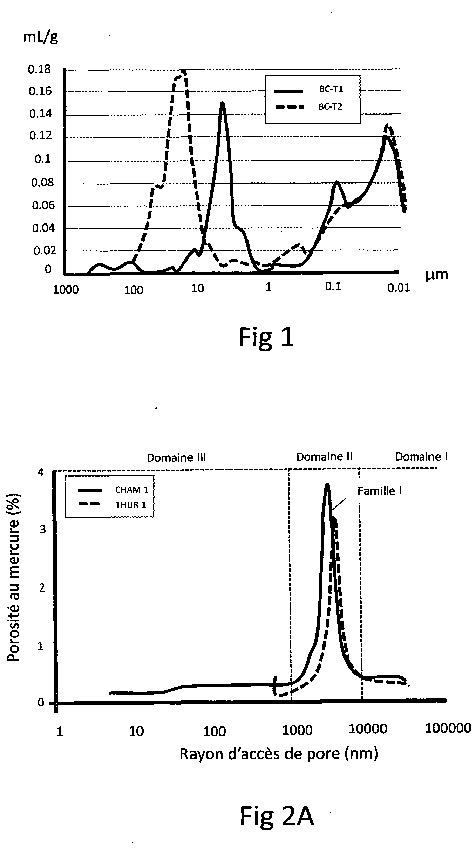

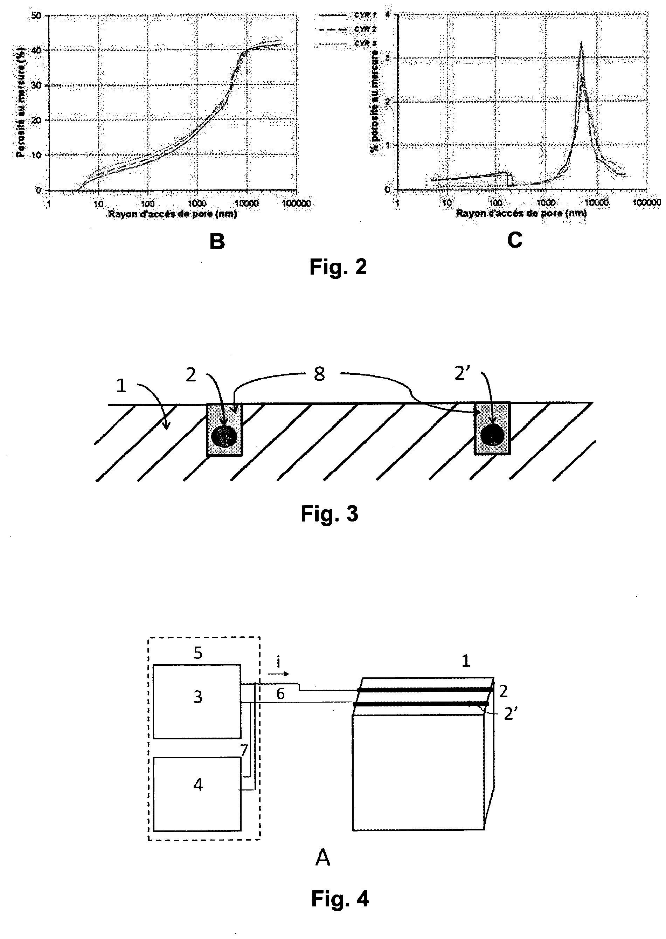

De manière encore plus préférentielle cette roche tendre crayeuse est du Tuffeau ayant une porosité est continue entre 3 et 30 000 nm. A titre indicatif, la Fig. 2 montre deux exemples de Tuffeaux satisfaisant aux critères de l'invention (D Dessandier, Thèse de l'Université de Tours « Étude du milieu poreux et des propriétés de transfert des fluides du Tuffeau blanc de Touraine », 23 juin 1995). Cela ne signifie pas que tous les Tuffeaux y satisfont, mais que la probabilité est grande de trouver les caractéristiques du matériau M parmi les Tuffeaux.

En plus des conditions précédentes, il est avantageux de choisir un matériau M qui ait une surface spécifique d'au moins 10m²/g. Parmi les Tuffeaux, cela se rencontre généralement parmi les roches contenant au moins 5% de smectite.

### Liste des figures

- la Fig. 1 reproduit la Figure 2 du document antérieur FR2 986 328
- la Fig. 2A extraite de la Thèse ci-dessus évoquée de D. Dessandier (p. 49) donne une première idée des distributions de pores habituelles dans le Tuffeau blanc ; les figures 2B et 2C (cf. Thèse p. 243) montrent les résultats de la porosimétrie au mercure de deux types particuliers de Tuffeau. En 2A on voit le nombre de pores pour chaque diamètre, et en 2B la proportion de pores ayant le diamètre considéré.
- la Fig. 3 montre un détail de l'implantation d'électrodes (2, 2') filaires dans le bloc (1) de matériau M, avec la présence du liant (8) réalisant le scellement.
- la Fig. 4A montre un mode de réalisation de l'invention où les électrodes (2, 2') sont scellées dans la même face du bloc (1) du matériau M, et la Fig. 4B un mode de réalisation où elles sont scellées dans deux faces opposées du bloc (1).
- la Fig. 5 montre un mode de réalisation de l'invention destiné à être immergé dans un matériau granulaire ou pulvérulent, et pouvant constituer un tensiomètre
- la Fig. 6A montre un mode de réalisation conforme à la figure 4A implantée dans un bac spécifique (10) pour plantes, afin de réaliser un monitoring hydrique de plantes, et la Fig. 6B est complétée de modules électroniques complémentaire rendant l'ensemble autonome (grâce à un remplissage en eau déclenché sur condition d'assèchement) et/ou communicant

### Description des réalisations préférentielles

Selon un premier mode de réalisation, le matériau M a été choisi parmi les Tuffeaux satisfaisant les caractéristiques de l'invention. Les électrodes sont au nombre de deux, rectilignes, constituées chacune d'une tige d'inox de 3 à 6 mm placé dans un sillon creusé dans le Tuffeau, d'un diamètre supérieur d'environ 1mm au diamètre de la tige d'inox et d'une profondeur de 1,5 à 2 fois ce diamètre, les deux tiges étant parallèles et placées dans des sillons parallèles conformément à la Fig. 3. On ne sortirait pas du cadre de l'invention en plaçant trois tiges parallèles, la première et la troisième étant reliées ensemble pour constituer une des électrodes (2), et la seconde constituant l'autre électrode (2').Le liant (8) du scellement est réalisé comme décrit ci-dessus par un mélange de poudre du matériau M et d'environ d'une proportion de chaux comprise entre 5 et 50% selon les performances recherchée.

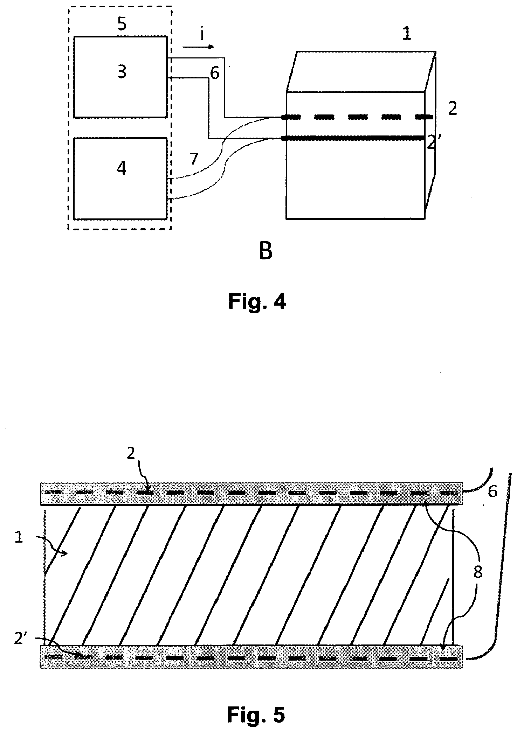

L'ensemble du dispositif selon l'invention est schématisé en Fig.4, selon deux modes de réalisation. Selon un premier mode de réalisation schématisé sur la Fig. 4A, l'invention comporte des électrodes (2, 2') scellées parallèlement côte à côte dans la même face du bloc (1) du matériau M, et connectées par une paire de conducteurs (6) à une électronique (3) apte à générer un courant de mesure. Elles sont choisies rectilignes, mais il va de soi qu'elles peuvent ne pas l'être sans pour autant que l'on sorte du cadre de l'invention. La tension portant l'information de résistance est alors prélevée par les conducteurs (7) l'amenant à une électronique (4) apte à réaliser cette mesure. L'ensemble (5) des électroniques (3) et (4) constitue un conditionneur électronique apte à mettre en œuvre les blocs (1) de matériau M portant au moins deux électrodes scellées, selon l'invention. Il va de soi que l'électronique (3) pourrait générer une tension constante, et l'électronique (4) mesurer le courant qui en résulte compte tenu de la résistance entre les électrodes (2, 2'). Une telle configuration équivaut à la précédente pour l'homme du métier.

Selon un second mode de réalisation schématisé sur la Fig. 4B, l'invention comporte des électrodes (2, 2') rectilignes scellées parallèlement comme ci-dessus, mais dans deux faces opposées du bloc (1) du matériau M. Elles sont a priori disposées en vis-à-vis de manière à minimiser l'épaisseur de matériau M que le courant aura à traverser, mais on pourrait aussi bien disposer en quinconce plusieurs paires ou un nombre impair d'électrodes. Les autres éléments portant les mêmes repères sont identiques au premier mode de réalisation, et ne seront donc pas répétés.

Selon une variante de ce second mode, que l'on appellera le troisième mode de réalisation, le bloc (1) est aminci dans la dimension qui sépare les faces portant les électrodes. A titre indicatif, cette dimension est généralement dans une fourchette comprise entre quelques mm et sensiblement quatre cm, selon la gamme de mesures qu'on veut réaliser. Lesdites électrodes (2,2') sont alors des grilles inoxydables laissant passer l'humidité, scellées parallèlement sur les deux faces opposées du bloc (1) par le liant (8) déjà évoqué ci-dessus, qui permet la conduction électrique et le passage de l'humidité. L'ensemble forme alors une sorte de sandwich apte à être immergé dans un matériau pulvérulent ou granuleux dont on veut mesurer l'humidité, sous réserve que ce matériau soit bien en contact avec le bloc (1) et qu'on l'y ait laissé un temps suffisant pour que l''équilibre hydrique soit établi.

Plongée dans un substrat de culture, une telle réalisation selon ce troisième mode de l'invention constitue un tensiomètre pour mesurer la teneur en eau des sols.

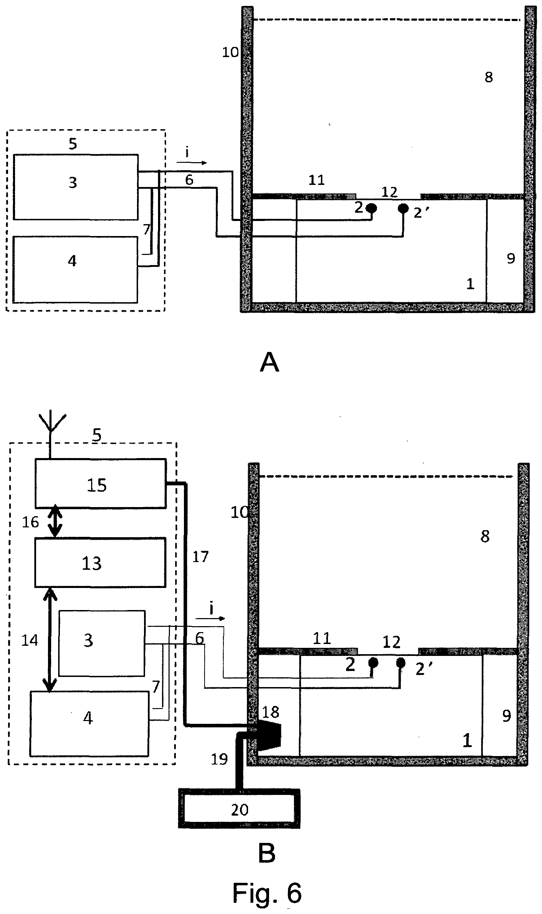

Selon une variante du premier mode de réalisation, qui sera appelée le quatrième mode de réalisation, un dispositif selon la Fig. 4A est incorporé dans un bac à plantes à réserve d'eau, conforme à l'objet de la demande de brevet FR 2 988 263 du 24/04/12 et délivré en octobre 2016. Le résultat est schématisé en Fig. 6A. Un tel bac (10) comporte un compartiment supérieur apte à recevoir de la terre ou un substrat (8) de culture, séparé par un fond (11) d'un compartiment inférieur (9) apte à recevoir une réserve d'eau. Un bloc (1) du matériau M permet à l'eau de remonter par capillarité lorsqu'il en existe dans la réserve (9), pour être distribuée aux racines des plantes passant par un trou (12) ménagé dans la plaque (11). Dans une seconde phase commençant lorsque cette réserve est tarie, le bloc (1) se vide d'une manière qui varie dans le temps et selon la tension de succion des plantes mises en place dans le haut du substrat (8). Cette manière est très proche du comportement hydrique d'une pleine terre parfaite, ce qui place progressivement le métabolisme de ces plantes en mode d'économie d'eau.

On peut aussi définir ce mode de réalisation comme un dispositif de mesure selon le premier mode de réalisation où les électrodes sont parallèles côte à côte sur une même face du bloc (1), où ledit bloc (1) est incorporé au fond d'un bac (10) à plantes comportant un compartiment supérieur apte à recevoir un substrat (8) de culture, séparé, par un fond (11) percé d'un trou (12), d'un compartiment inférieur (9) apte à recevoir une réserve d'eau, caractérisé en ce que le bloc (1) du matériau M est apte à faire remonter par capillarité l'eau qui peut se trouver dans la compartiment inférieur (9) pour la distribuer par le trou (12) à des plantes situées dans le substrat (8), et caractérisé en ce que les électrodes (2, 2') sont disposées en travers de toute circulation d'eau du bloc (1) vers le trou 12.

L'implantation de l'invention dans le bloc (1) du matériau M réalisant cette double fonction d'hydratation est extrêmement avantageuse et permet le monitoring de la (ou les) plante(s). Un tel dispositif peut être complété par une intelligence embarquée et évoluer vers un objet autonome et/ou communicant schématisé en Fig. 6B. Une intelligence embarquée dans un circuit (13) à microcontrôleur permet d'analyser la situation hydrique et de générer des actions qui autonomisent le fonctionnement. Par exemple, sur critère de sécheresse, l'activation via un câble (17) d'une pompe à eau (18) permettant de remplir à nouveau la réserve d'eau via un tuyau (19) plongeant dans une réserve d'eau déportée (20), ce qui rend cet ensemble autonome en eau. Enfin il est aisé pour l'homme du métier d'ajouter à cela un système communicant (15) via un bus de données (16) relié au circuit (13). Ce circuit communicant est schématisé avec une antenne pour symboliser son côté communicant, mais en réalité l'antenne est intégrée dans le module électronique (15) : pour cette raison elle ne porte aucun repère sur le dessin. Cette communication peut se faire à distance par Bluetooth, Wifi ou au moyen d'une carte Sim, afin de constituer un objet communicant apte à informer un opérateur à distance et/ou recevoir dudit opérateur une consigne de remplissage.

## Revendications

1. Dispositif de mesure résistive de l'humidité d'un matériau minéral poreux, comprenant :
- un bloc (1) d'un matériau M minéral, poreux et rigide, dont on veut mesurer les variations de résistance en fonction de la teneur en eau
- au moins deux électrodes (2) et (2') rigides inoxydables appliquées parallèlement contre ledit matériau M, et scellées sur ce dernier par une couche de liant (8) constitué d'un mélange de poudre du matériau M et de chaux,
chacune des électrodes (2) et (2') étant connectée à :
- une électronique (3) apte à générer un courant i de mesure et
- une autre électronique (4) apte à mesurer la résistance entre lesdites électrodes. »

2. Dispositif de mesure selon la revendication 1, **caractérisé en ce que** le matériau M est un matériau naturel ayant une porosité ouverte dont la distribution des diamètres est continue entre 10 et 10 000 nm.

3. Dispositif de mesure selon la revendication 2, **caractérisé en ce que** le matériau M est un Tuffeau, et **en ce que** la couche de liant (8) est constituée de chaux dans une proportion comprise entre 5 et 50% et de poudre du matériau M.

4. Dispositif de mesure selon l'une quelconque des revendications 2 ou 3, **caractérisé en ce que** le matériau M possède une surface spécifique au moins égales à 10m²/g.

5. Dispositif de mesure selon la revendication 2 ou 3, **caractérisé en ce que** les électrodes (2, 2') sont rectilignes et scellées parallèlement côte à côte sur une même face du bloc (1) de matériau M.

6. Dispositif de mesure selon la revendication 2 ou 3, **caractérisé en ce que** les électrodes (2, 2') sont scellées parallèlement sur deux faces opposées du bloc (1) de matériau M, **en ce que** ledit bloc est aminci dans la dimension qui sépare ces faces portant les électrodes, et **en ce que** lesdites électrodes (2,2') sont des grilles inoxydables laissant passer l'humidité tout comme la couche de liant (8).

7. Ensemble comportant un dispositif de mesure selon la revendication 5 incorporé au fond d'un bac (10) à plantes comportant un compartiment supérieur apte à recevoir un substrat (8) de culture, séparé, par un fond (11) percé d'un trou (12), d'un compartiment inférieur (9) apte à recevoir une réserve d'eau, **caractérisé en ce que** le bloc (1) du matériau M est apte à faire remonter par capillarité l'eau qui peut se trouver dans la compartiment inférieur (9) pour la distribuer par le trou (12) à des plantes situées dans le substrat (8), et **caractérisé en ce que** les électrodes (2, 2') sont disposées en travers de toute circulation d'eau du bloc (1) vers le trou 12.

8. Ensemble selon la revendication 7 ayant en outre des moyens électroniques le rendant plus autonome en eau et/ou communicant.

## Patentansprüche

1. Ohm'sche Messvorrichtung der Feuchtigkeit eines porösen, mineralischen Materials, umfassend:
- einen Block (1) eines porösen, mineralischen und steifen Materials M, dessen Widerstandsabweichungen in Abhängigkeit von dem Wassergehalt gemessen werden sollen
- wenigstens zwei steife, rostfreie Elektroden (2) und (2'), die parallel gegen das genannte Material M angewendet sind und auf diesem durch eine Bindemittelschicht (8) versiegelt sind, die aus einer Pulvermischung des Materials M und Kalk gebildet ist,
wobei jede der Elektroden (2) und (2') angeschlossen ist an:
- eine Elektronik (3), die geeignet ist, einen Messstrom i zu erzeugen und
- eine andere Elektronik (4), die geeignet ist, den Widerstand zwischen den genannten Elektroden zu messen."

2. Messvorrichtung gemäß Anspruch 1, **dadurch gekennzeichnet, dass** das Material M ein natürliches Material ist, das eine offene Porosität aufweist, deren Verteilung der Durchmesser zwischen 10 und 10.000 nm kontinuierlich ist.

3. Messvorrichtung gemäß Anspruch 2, **dadurch gekennzeichnet, dass** das Material M ein Tuffstein ist und dass die Bindemittelschicht (8) aus Kalk, die in einem Anteil aus Kalk zwischen 5 und 50 % inbegriffen ist, und aus Pulver des Materials M gebildet ist.

4. Messvorrichtung gemäß irgendeinem der Ansprüche 2 oder 3, **dadurch gekennzeichnet, dass** das Material M eine spezifische Oberfläche besitzt, die mindestens gleich 10 m²/g ist.

5. Messvorrichtung gemäß Anspruch 2 oder 3, **dadurch gekennzeichnet, dass** die Elektroden (2, 2') geradlinig und parallel Seite an Seite auf einer und derselben Seite des Blocks (1) aus Material M sind.

6. Messvorrichtung gemäß Anspruch 2 oder 3, **dadurch gekennzeichnet, dass** die Elektroden (2, 2') parallel auf zwei entgegengesetzten Seiten des Blocks (1) aus Material M versiegelt sind, dass der genannte Block in der Abmessung verdünnt ist, die diese, die Elektroden tragenden Seiten trennt, und dass die genannten Elektroden (2, 2') rostfreie Gitter sind, die die Feuchtigkeit wie ebenso die Bindemittelschicht (8) hindurchtreten lassen.

7. Gruppe, umfassend eine Messvorrichtung gemäß Anspruch 5, die am Boden eines Pflanzkübels (10) eingelassen ist, umfassend ein oberes Fach, das geeignet ist, ein Zuchtsubstrat (8) aufzunehmen, das durch einen mit einem Loch (12) durchbohrten Boden (11) von einem unteren Fach (9) getrennt ist, das geeignet ist, einen Wassertank aufzunehmen, **dadurch gekennzeichnet, dass** der Block (1) des Materials M geeignet ist, per Kapillarwirkung das Wasser wieder hochzuziehen, das in dem unteren Fach (9) angeordnet sein kann, um es durch das Loch (12) an die Pflanzen zu verteilen, die in dem Substrat (8) angeordnet sind, und **dadurch gekennzeichnet, dass** die Elektroden (2, 2') über den gesamten Wasserkreislauf des Blocks (1) zu dem Loch (12) angeordnet sind .

8. Gruppe gemäß Anspruch 7, die darüber hinaus elektronische Mittel aufweist, die sie in Bezug auf Wasser autonomer und/oder zusammenhängender macht.

## Claims

1. Device for resistive measurement of the moisture of a porous material, comprising:
- a block (1) of a material M which is mineral, porous and rigid, of which it is desired to measure the resistance variations as a function of the water content
- at least two electrodes (2) and (2') which are rigid and non-oxidising, applied parallel against the said material M, and sealed on to the latter by a binder (8) consisting of a blend of powder of material M and lime,
where each electrode (2) and (2') is connected to an electronic unit (3) able to generate a measuring current i and another electronic unit (4) able to measure the resistance between the said electrodes.

2. Measuring device according to claim 1, **characterised in that** material M is a natural material with open porosity, the distribution of the diameters of which is continuous between 10 and 10,000 nm (0.01 to 10 µm).

3. Measuring device according to claim 2, **characterised in that** material M is a Tuffeau, and **in that** the binder (8) consisting of lime is in a proportion of between 5 and 50% and powder of material M.

4. Measuring device according to either of claims 2 or 3, **characterised in that** material M has a surface specific area at least equal to 10 m²/g.

5. Measuring device according to claim 2 or 3, **characterised in that** the electrodes (2, 2') are rectilinear and sealed parallel side-by-side on to the same side of the block (1) of material M.

6. Measuring device according to claim 2 or 3, **characterised in that** the electrodes (2, 2') are sealed parallel on to two opposite sides of the block (1) of material M, **in that** the said block is made thinner in the dimension separating these sides bearing the electrodes, and **in that** the said electrodes (2, 2') are non-oxidising grids allowing the moisture and the binder (8) to pass through.

7. Assembly including a measuring device according to claim 5 incorporated in the base of a plant container (10) including an upper compartment which can contain a culture substrate (8), separated, by a base (11) with a hole (12), from a lower compartment (9) which can contain a water reserve, **characterised in that** the block (1) of material M is able to raise by capillarity any water in the lower compartment (9) to distribute it through the hole (12) to plants in the substrate (8), and **characterised in that** the electrodes (2, 2') are positioned perpendicular to any water flow from the block (1) towards hole (12).

8. Assembly according to claim 7 also having electronic means making it more water self-sufficient and/or communicating.
